# EUROPEAN PATENT SPECIFICATION

(11) **EP 4 173 618 B1**
(45) Date of publication and mention of the grant of the patent: **23.09.2026**
(21) Application number: 22204330.9
(22) Date of filing: 28.10.2022
(51) Int. Cl.: A61K 31/135, A61K 31/16, A61K 31/4412, A61K 31/714, A61K 33/00, A61K 33/04, A61K 33/24, A61K 38/06, A61K 38/42, A61P 39/02, A61K 31/4196

(54) **COMPOSITION FOR UPPER RESPIRATORY TRACT ADMINISTRATION COMPRISING A METAL CHELATOR AND A CYANIDE ANTIDOTE AND METHOD THEREOF**
ZUSAMMENSETZUNG ZUR VERABREICHUNG IN DIE OBEREN ATEMWEGE, DIE EINEN METALLCHELATATOR UND EIN CYANID-GEGENMITTEL UMFASST, UND VERFAHREN DAFÜR
COMPOSITION POUR L'ADMINISTRATION DES VOIES RESPIRATOIRES SUPERIEURES COMPRENANT UN CHELATEUR DE METAL ET UN ANTIDOTE DE CYANURE ET PROCEDE ASSOCIE

(30) Priority: 29.10.2021 US 202163273317 P
(43) Date of publication of application: 03.05.2023
(73) Proprietor: Original Biomedicals Co., Ltd., Tainan City 744 (TW)
(72) Inventor: Chen, Jia-Long, 744 Tainan City (TW); Liao, Wei-Chuan, 744 Tainan City (TW); Sun, Tzu-Hui, 744 Tainan City (TW); Chen, Chia-Hung, 744 Tainan City (TW); Wang, Chau-Hui, 744 Tainan City (TW); Yen, Hsiao-Pao, 744 Tainan City (TW)
(74) Representative: Cabinet Chaillot

(56) References cited:
- LALONDE C ET AL: "AEROSOLIZED DEFEROXAMINE PREVENTS LUNG AND SYSTEMIC INJURY CAUSED BY SMOKE INHALATION", JOURNAL OF APPLIED PHYSIOLOGY, AMERICAN PHYSIOLOGICAL SOCIETY, US, vol. 77, no. 5, 1 November 1994 (1994-11-01), pages 2057 - 2064, XP000972532, ISSN: 0021-8987
- MAYNARD R L: "William Harvey and air pollution", OCCUPATIONAL AND ENVIRONMENTAL MEDICINE, vol. 60, no. 2, 1 February 2003 (2003-02-01), pages 147 - 147, XP093030086, ISSN: 1351-0711, DOI: 10.1136/oem.60.2.147
- ESTOURGIE-VAN BURK G FREDERIEK ET AL: "Intranasal treatment of vitamin Bdeficiency in children", EUROPEAN JOURNAL OF PEDIATRICS, SPRINGER BERLIN HEIDELBERG, BERLIN/HEIDELBERG, vol. 179, no. 2, 22 November 2019 (2019-11-22), pages 349 - 352, XP036993012, ISSN: 0340-6199, [retrieved on 20191122], DOI: 10.1007/S00431-019-03519-0
- MISCHLEY LAURIE K ET AL: "Central nervous system uptake of intranasal glutathione in Parkinson’s disease", vol. 2, no. 1, 25 February 2016 (2016-02-25), pages 1 - 6, XP055824320, Retrieved from the Internet <URL:https://www.nature.com/articles/npjparkd20162.pdf> DOI: 10.1038/npjparkd.2016.2

## Description

### FIELD OF THE INVENTION

The present invention relates to a composition for upper respiratory tract administration comprising a cyanide antidote and a metal chelator, and the composition is for use in the cure or protection or prophylaxis of fire injury.

### BACKGROUND

In a fireground, heavy smoke deriving from completely burnt and incompletely burnt items contains solid smoke particles, toxic gases and a large amount of smog generated by splitting decomposition at high temperature. The heavy smoke can severely damage health of firefighters, on-site victims or residents in the neighborhood.

In recent years, species and amounts of toxic gases in a fireground have been dramatically increased because synthetic building materials and polymer materials are widely used in house furnishing, offices and public places of entertainment. For example, the toxic gases include acrylicin, acrylonitrile, benzene, formaldehyde, sulfur dioxide, hydrogen cyanide, dioxin, polycyclic aromatic hydrocarbons, etc.. These toxic gases can directly or indirectly cause irreversible damages such as illness, degeneration, or chronic disease in a personnel. Amongst these toxic gases, hydrogen cyanide (HCN) is an instant toxic substance. HCN demonstrates high affinity to ferric ions in cytochrome oxidases. When HCN enters a human body, it forms stable complex with cytochrome oxidases and interferes with oxygen binding cytochrome oxidases, which eventually results in inaccessibility of oxygen to the body cells.

Kelocyanor^{®} having dicobalt edetate is an injective form cyanide antidote. Cobalt ions form stable complex with cyanide in the blood, and the complex is excreted out of the body via urine. However, cobalt exists in an ionic form remains unpredictably toxic to an individual.

Disclosed in Pat. No. GB1404324 is a process for the diagnosis of chronic hypercyanogenesis. A composition having sodium thiosulphate and hydroxocobalamine is used to track the variating trend of CN⁻ concentration in the blood. The composition is administered at a single dose or multiple doses via intramuscular injection. Nonetheless, hypercyanogenesis deteriorates after multiple-dose administration, which implicates its inapplicability to chronic therapy.

Disclosed in Pat. No. US5834448 is a new dosage form of hydroxocobalamin and its antidotal use in cyanide poisoning. The hydroxocobalamin is freeze-dried in an acidic medium so that it can be instantly redissolved in a neutral saline solution. Stability of the hydroxocobalamin in preservation is significantly improved. However, the hydroxocobalamin-based pharmaceutical compositions provided therein is administered via intravenous injection. The dosage form limits its applicability in an environment where cyanide exposure reaches hazardous level. The hydroxocobalamin-based pharmaceutical compositions can not be administered directly into the environment or to subjects in need by inhalation to achieve protective or therapeutic effect.

In addition to toxic gases as previously described, heavy smoke in a fireground further include particulate matters, dust, fibers, glass fibers or other hazardous substances. These hazardous substances is not only life-threatening to on-site disaster relief workers, but also detrimental to post-disaster inspectors. During post-disaster inspection, atmosphere at fireground would be full of smoke substances such as dust, particulate matters or fibers when fire debris is moved, which may harm post-disaster inspectors' health when they are exposed to or even inhale those hazardous substances.

According to "Standard Operating Procedures for Fire Investigation and Identification" issued by the Fire Department of the Ministry of the Interior, R.O.C., inspectors must be equipped with antigas cartridges when entering a fireground for post-disaster investigation. The antigas cartridge can efficiently filter most of toxic gases such as organic gases, chlorine, hydrochloric acid, sulfur dioxide, hydrogen sulfide, or chlorine dioxide. Nevertheless, in practical use, it requires a long time for antigas cartridge to filter toxic gases such as hydrogen chloride, hydrogen cyanide and acrolein, which limits its protective effect and also increases risk of inhaling toxic gases by personnel.

On top of using antigas cartridge, current means of protecting personnel in exposure to hazardous substances in a fireground further includes physical isolation such as wearing respiratory system protective equipment. However, heavy smoke in a fireground is a mixture of particulate matters and toxic gases, and the averaged particle size is less than 10 um. Hence, current physical protective equipment remains unable to completely filter particulate matters in fireground atmosphere. Meanwhile, solid microparticles in the heavy smoke are irritative to upper respiratory tract and cause an abundance of mucous secretion which may congest the upper respiratory tract.

Withal, in a fireground, inhalation of hydrogen cyanide and free radicals from splitting decomposition is also considered a main cause of instantaneous injury in that free radicals leads to strong oxidative stress to human body tissues. When abundance of internal free radicals exceeds a normal range, "free radical chain reaction" starts to oxidize proteins, carbohydrates or lipids and new free radicals are generated thereby. Excessive free radicals bring damage to genetic substances (such as DNA), lipid peroxidation, enzyme deactivation, and inflammation due to abnormal activity of immune cells including monocytes or macrophages. "Free radical chain reaction" jeopardizes an individual's health from both a short-term and a long-term perspective.

With regard to this, to overcome the dilemma that current technology can not guarantee protection against both solid and gaseous hazardous substances in fireground smoke, development of a novel means to eliminate and to neutralize both kinds of hazardous substances is urgently required. The novel means does not only protect firefighters' health and prevent them from heavy smoke damage, but also reverse damage resulting from inhalation of the heavy smoke, and injuries are effectively cured thereupon.

LALONDE C ET AL: "AEROSOLIZED DEFEROXAMINE PREVENTS LUNG AND SYSTEMIC INJURY CAUSED BY SMOKE INHALATION", JOURNAL OF APPLIED PHYSIOLOGY, AMERICAN PHYSIOLOGICAL SOCIETY, US, vol. 77, no. 5, 1 November 1994 (1994-11-01), pages 2057-2064, XP000972532, ISSN: 0021-8987 assessed the role of oxidant release at the airway mucosal surface on airway injury and systemic response to a severe smoke insult. Adult sheep (n = 20) were insufflated with well-characterized smoke from burning cotton toweling. A standardized dose of 12 breaths of smoke with a tidal volume of 20 ml/kg was given under anesthesia. Sheep were awakened, monitored for 24 h, and killed; data were compared with control sheep. Sheep were given 1) humidified oxygen, 2) continuous aerosol of 10% deferoxamine (DFO)-pentastarch solution beginning after smoke, 3) DFO-alone aerosol, or 4) pentastarch-alone aerosol. DFO has antioxidant properties directly and chelates iron. Severe respiratory failure occurred in all but DFO-pentastarch group. Shunt fraction increased from a control of 4%. Histological assessment revealed severe airway mucosal edema, ulceration, bronchorrhea, and severe atelectasis but only moderate alveolar edema. Increased lipid peroxides were also noted in free airway fluid and in bronchoalveolar lavage fluid. In addition, oxygen consumption increased by 75%, fluid requirements increased threefold, and protein-rich systemic soft tissue lymph flow doubled, all significant increases compared with control sheep. No significant physiological or histological changes were noted in DFO-pentastarch aerosol group. We conclude that 1) oxidants possibly initiated through free iron release are involved in severe smoke-induced airway injury and resulting systemic inflammatory response, probably through an amplified oxidant injury and 2) an aerosol of a DFO-pentastarch complex prevents the injury process, whereas DFO alone is not effective as an aerosol. (J Appl Physiol (1985). 1994 Nov;77(5):2057-64).

### SUMMARY

The invention is set out in the appended set of claims.

Considering that heavy smoke in a fireground is a complicated damage form to on-site personnel's life and safety, the instant inventor manages to develop a new type of means for comprehensive protection and treatment in addition to current means of hydrogen cyanide elimination. The present invention aims to provide a means for elimination and neutralization of both solid and gaseous hazardous substances in heavy smoke. In view of this, the present invention provides a composition for upper respiratory tract administration comprising a cyanide antidote and a metal chelator, and the composition is used for cure or protection from fire injury.

In various embodiments, the cyanide antidote is selected from a group consisting of hydroxocobalamin, dicobalt edetate, cobinamide, aquohydroxocobinamide, dinitrocobinamide, methemoglobin, sodium nitrite, amyl nitrite, dimethyl aminophenol, sodium thiosulfate and glutathione.

In various embodiments, the metal ion chelator is selected from a group consisting of deferoxamine, deferiprone and deferasirox.

In some embodiments, the composition further comprises an expectorant selected from a group consisting of KI, iodinated glycerol, glyceryl guaiacolate, guaifenesin, ambroxol, bromhexine, N-acetylcysteine and lysozyme.

In some embodiments, the composition further comprises a carrier, wherein the carrier comprising ionized water, secondary water, ultra-pure water or buffer solution. Preferably, the buffer solution having a buffer solute selected from a group consisting of phosphate, tris(hydroxymethyl)aminopropanesulfonic acid, dihydroxyethylglycine, tris(hydroxymethyl)aminomethane, tris(hydroxymethyl)methylglycine, 4-hydroxyethylpiperazineethanesulfonic acid acid, n-tris(hydroxymethyl)methyl-2-aminoethanesulfonic acid, 3-(n-morpholine)ethanesulfonic acid, piperazine-n,n'-bis(2-ethanesulfonic acid), dimethylarsinic acid, sodium citrate and 2-morpholineethanesulfonic acid

In various embodiments, the composition is further used for protection and cure confronting with potential exposure to an environment of high concentration cyanide.

Additional embodiments of the present invention disclose a method for upper respiratory tract administration comprising administrating the aforementioned composition to a subject in need at a time point until the composition reaches an effective dose so as to suppress injury upon the subject in need in exposure to toxic gases, wherein the time point is prior to or post exposing the subject in need to the toxic gases.

In various embodiments, the toxic gases comprising particulate matters, free radicals or hydrogen cyanide.

Preferably, the route of administration comprises biological fluid absorption or mucosal absorption.

Preferably, the subject in need comprises a mammal.

Current means to eliminate hydrogen cyanide relies on solution injection as a main delivery form, such as intramuscular injection or intravenous injection. It requires time for effects to emerge, and instability of the dosage forms is also concerned, which insuperably obstructs user end as well as producer end. Besides, cyanide poisoning is not the only risk in a fireground, other harmful factors including particulate matters and free radicals are also difficult for prior technologies to overcome.

Places having high tendency of cyanide poisoning includes workplaces of particular occupations, such as electroplating, metallurgy, plastic industry. Besides acute toxication, long-term exposure of a personnel in an environment of potential cyanide poisoning is also taken into considerations. Wherefore, drug administration regards not only to treatment in acute poisoning phase, but also to precaution of poisoning prior to personnel's entry into these workplaces. Prior technologies with injection dosage forms remain ineffectual in the above situations.

The composition for upper respiratory tract administration and the method thereof in the present invention provides solutions for curing and prevention of acute or chronic cyanide poisoning, addressing issues of inexpedient cyanide poisoning treatment encountered by prior arts. Additionally, metal ion chelator blocks free radical chain reaction, and relieves oxidative stress of tissues and cells in a human body. Expectorant promotes mucosal excretion to eliminate particulate matters' irritation and jamming in upper respiratory tract so that the upper respiratory tract maintains unobstructed.

In sum, the composition for upper respiratory tract administration and the method thereof in the present invention overcome limitations of dosage forms in prior technologies. The composition rapidly enters a body at a low dose and achieves protective and therapeutic effect in a normal situation, fireground or environment of trace cyanides.

Furthermore, the composition for upper respiratory tract administration is not limited to on-site environment or subject's status during administration. The composition is nebulized for an subject to inhale when necessary. The composition is absorbed via subject's lung and enters the blood circulation, or forms a protective layer on the surface of upper respiratory tract and alveoli. Both fire injury protection and treatment can be achieved, and first aid of acute poisoning as well as prevention in long-term exposure are both taken into account.

### BRIEF DESCRIPTIONS OF DRAWINGS

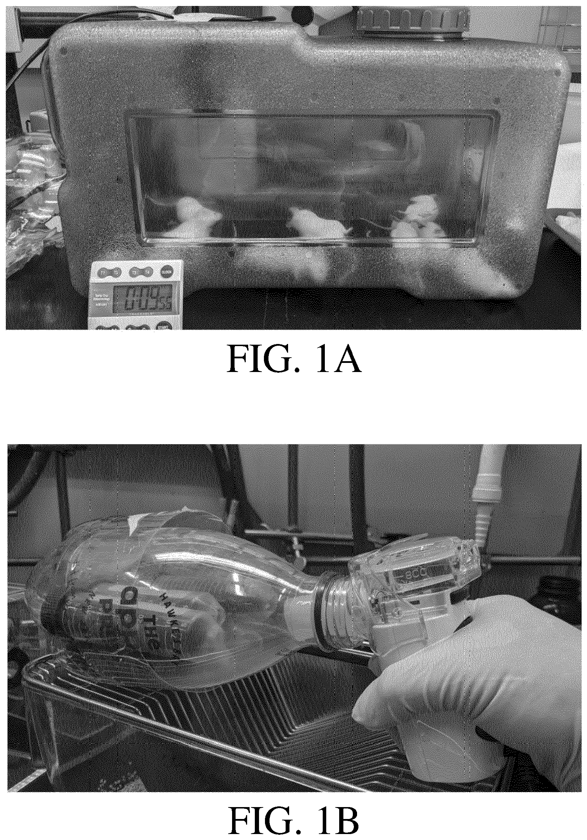

Fig. 1A illustrates an exemplary environment of gaseous cyanide in a barrel. Fig. 1B illustrates an exemplary administration of the composition for upper respiratory tract in a nebulized form.

### DETAILED DESCRIPTION

The present disclosure provides a composition for upper respiratory tract administration comprising a cyanide antidote and a metal chelator, and the composition is used for cure or protection from fire injury; preferably, the composition comprises 5 to 10 parts by weight cyanide antidote, and 1 to 5 parts by weight metal ion chelator.

The cyanide antidote is selected from a group consisting of hydroxocobalamin, dicobalt edetate, cobinamide, aquohydroxocobinamide, dinitrocobinamide, methemoglobin, sodium nitrite, amyl nitrite, dimethyl aminophenol, sodium thiosulfate and glutathione. Preferably, the cyanide antidote comprises hydroxocobalamin. Concretely, the cyanide antidote forms complexes with cyanide ion via ligand binding so as to neutralize toxicity of cyanide ions. Competition of the cyanide ions with oxygen for divalent ferric ions (Fe²⁺) is avoided thereby. For example, hydroxocobalamin, a.k.a. vitamin B12a, has a cobalt ion at its chemical structural center. The cobalt ion binds to cyanide ions (CN⁻) and cyanocobalamin, a.k.a. vitamin B12, is formed. Cyanocobalamin is detoxified and excreted from the body along with water. Meanwhile, hydroxocobalamin rapidly enters mitochondria and binds to cyanides so that cellular oxidative metabolism is restored.

The metal ion chelator is selected from a group consisting of deferoxamine, deferiprone and deferasirox. Preferably, the metal ion chelator comprises deferoxamine. Specifically, a large amount of free radicals are generated by splitting decomposition at high temperature in a fireground. Fenton's reaction initiates after the free radicals are inhaled into the body and react with internal metal ions, such as ferric ions or copper ions. Fenton's reaction produces more free radicals and causes more oxidative stress over tissues and cells, which eventually leads to free radical damage. To obviate free radical damages, the metal ion chelator in the composition, such as deferoxamine, competes with free radicals for ferric ions in blood stream. Thus, the free radical chain reaction is blocked and cells are protected from oxidative stress.

In additional embodiments, to remove excessive mucous secreted by the irritated upper respiratory tract when a subject inhales hazardous substances such as particulate matters, the composition further comprises an expectorant selected from a group consisting of KI, iodinated glycerol, glyceryl guaiacolate, guaifenesin, ambroxol, bromhexine, N-acetylcysteine and lysozyme. Preferably, the expectorant comprises N-acetylcysteine. More preferably, the composition comprises 5 to 10 parts by weight cyanide antidote, 1 to 5 parts by weight metal ion chelator and 5 to 10 parts by weight expectorant.

Particularly speaking, an expectorant breaks disulfide bonds to lower mucous viscosity so that excretion of sputum is promoted. For instance, N-acetylcysteine has a thiol group (-SH) whose sulfur atom possesses a larger 3s/3p hybridized orbital, and hydrogen atom has a smaller 1s orbital. Therefore, S-H bond is weak and tends to be oxidized, and the lone pair on sulfur atom is unmasked to reduce and break disulfide bond, which reduces viscosity of sputum. Bromhexine stimulates bronchial mucous secretion and further dissolves the mucous so as to diminish mucous viscosity. Also, pseudostratified columnar epithelium is activated by bromhexine to drive the mucous out of the body. Regarding to ambroxol, as an active metabolite of bromhexine, ambroxol presents similar characteristics of mucous elimination and secretion dissolution. Ambroxol is able to accelerate mucous excretion out of respiratory tract and minimize the amount of remaining mucous. Amborxol enhances removal of sputum so that respiration is improved. Likewise, lysozyme is a native antibacterial enzyme existing in human secretion such as tears or saliva. Carbon-oxygen bond of the substrate is broken by side chains of Glu35 and Asp52 in lysozyme protein sequence, and mucous is then decomposed so that excretion of sputum is promoted. In addition to mucous decomposition, other expectorants achieves at rapid sputum excretion through stimulating cells on respiratory tract surface, such as respiratory epithelium cells. For example, glyceryl guaiacolate directly excites bronchial secretory cells (e.g. Clara cell) and the subject's reflex to dilute sputum.

In the present disclosure, the composition further comprises a carrier so as to transport the composition into a human body in a variety of dosage forms. The dosage form is a spray, an inhaler, a nebulizer, or drops. The inhaler is a metered-dose inhaler, a spray inhaler or a dry powder inhaler. The carrier may be a solvent or solution with appropriate solubility for the active ingredient, but not limited to this. In various embodiments, the carrier comprises ionized water, secondary water, ultra-pure water or buffer solution. Preferably, the buffer solution has a buffer solute selected from a group consisting of phosphate, tris(hydroxymethyl)aminopropanesulfonic acid, dihydroxyethylglycine, tris(hydroxymethyl)aminomethane, tris(hydroxymethyl)methylglycine, 4-hydroxyethylpiperazineethanesulfonic acid acid, n-tris(hydroxymethyl)methyl-2-aminoethanesulfonic acid, 3-(n-morpholine)ethanesulfonic acid, piperazine-n,n'-bis(2-ethanesulfonic acid), dimethylarsinic acid, sodium citrate and 2-morpholineethanesulfonic acid

In various embodiments, the composition comprises 5 to 10 parts by weight the cyanide antidote, 1 to 5 parts by weight the metal ion chelator, 5 to 10 parts by weight the expectorant and 60 to 200 parts by weight the carrier.

Similarly, volatility of cyanides is harmful to on-site personnel in various occupational workplaces. Taking hydrogen cyanide for example, it tends to evaporate and dissolve in water at temperature above 28□. Hydrogen cyanide can be released by cyanogenetic glucoside. Cyanogenetic glucoside is a natural ingredient in some economical plants, such as bitter almonds, sorghum, cassava, Lima beans, drupe or bamboo shoots. For instance, root of cassava contains aminolevulinic acid dehydratase (ALAD) which enzymatically digests cyanogenetic glucoside and releases cyanoalcohol. Cyanoalcohol breaks up into hydrogen cyanide in low acidic environment, which eventually increases concentration of cyanides in atmosphere in a cassava-processing workplace. In view of this, the composition for upper respiratory tract administration in the present disclosure offers a solution for protection and treatment to personnel in the aforementioned workplaces where the personnel is potentially exposed to high concentration of cyanides, and minimizes occupational injury thereof. The workplaces can be exemplified by places for cassava processing, sorghum processing, or drupe processing, or further by places for photography, electroplating, hydrogen sulfide manufacturing, fumigant manufacturing, pharmaceutical manufacturing, papermaking, dyeing, rodenticide operation, insecticide operation, blast furnace gas operation, cyanide manufacturing, nitric acid manufacturing, rubber plastic manufacturing, synthetic fiber manufacturing, leather manufacturing, pesticide industry, gold and silver processing, metal surface hardening, Prussian blue fiber printing, metallurgical operation, bone phosphoric acid extraction, organic nitrogen compound manufacturing, soda manufacturing, gold inlay operation, biochemical weapons manufacturing, lighting gas manufacturing, or nitrocellulose burning operations, but not limited to this.

Several examples and experimental examples are listed below to describe the embodiments of the present invention and their technical effects in more detail, but the present invention is not limited by them.

### Test of mouse survival rate

A barrel having volume of 25 liters was prepared, and a tech foam was incinerated inside the barrel so as to produce gaseous cyanides. Then, 50 milliliters gaseous cyanides were withdrawn from the barrel by a syringe, and injected into a vacant bottle having volume of 1250 milliliters for dilution and concentration measuring. The actual cyanide concentration was calculated thereafter.

When cyanide concentration in the barrel reached 189.8±5.8ppm, 30 C57BL/6 mice were placed in the barrel, and 2 liters of 95% oxygen was pumped into the barrel for C57BL/6 mice basic oxygen consumption. Fig. 1A illustrates an example of cyanide exposure barrel in subsequent experimental examples. Please refer to TABLE 1, TABLE 1 illustrates test condition and preliminary test results of mice survival rate test, revealing the lethal dose of cyanide for C57BL/6 mice. On a condition of cyanide concentration at 189.8±5.8ppm, C57BL/6 mice were removed from the barrel at 20% death rate after inhaling cyanides for 30, 45, and 60 minutes. The corresponding survival rates were 62.5%, 37.5% and 0.0%, respectively.

**TABLE 1**

| **Strain** | **gender** | **age (week)** | **Body weight (g)** | **Cyanide con. (ppm)** | **Exposure time (min)** | **Total number** | **Survival number** | **Survival rate (%)** |
|---|---|---|---|---|---|---|---|---|
| **C57BL/6** | Male | 8 to 9 | 21.7 ±0.2 | 189.8 ±5.8 | 60 | 10 | 0 | 0.0 |
| | | | | | 45 | 16 | 6 | 37.5 |
| | | | | | 30 | 15 | 7 | 62.5 |
| **ICR** | male | 8 to 9 | 30.6 ±0.2 | 302.8 ±10.7 | 10 | 6 | 0 | 0.0 |
| | | | | | 7.5 | 8 | 3 | 37.5 |
| | | | | | 5 | 16 | 10 | 46.7 |

On the other hand, survival rate of ICR mice was also assessed. When cyanide concentration in the barrel reached 302.8±10.7ppm, 41 ICR mice were placed into the barrel, and 2 liters of 95% oxygen was pumped into the barrel for ICR mice basic oxygen consumption. Please continue to refer to TABLE1, the lower columns illustrate test conditions and preliminary test results of mice survival rate test, revealing the lethal dose of cyanide for ICR mice. On a condition of cyanide concentration at 302.8±10.7ppm, ICR mice were removed from the barrel at 20% death rate after inhaling cyanides for 5, 7.5, and 10 minutes. The corresponding survival rates were 46.7%, 37.5% and 0.0%, respectively.

### Example 1

A composition for upper respiratory tract administration was prepared by mixing 1 ml distilled water, 25 mg hydroxocobalamin and 1 mg deferoxamine.

### Example 2

A composition for upper respiratory tract administration was prepared by mixing 1 ml distilled water, 125 mg hydroxocobalamin and 5 mg deferoxamine.

### Comparative 1

Hydroxocobalamin water solution was prepared by mixing 1 ml distilled water and 25 mg hydroxocobalamin.

### Comparative 2

Deferoxamine water solution was prepared by mixing 1 ml distilled water and 5 mg deferoxamine.

### Experimental example 1

In the experimental example 1, survival rates of control and experimental groups of C57BL/6 mice were compared. The mice were exposed in an environment containing 184 to 189.8ppm cyanide for 24 to 39 minutes. The increase of mice survival rates corresponding to each treatment of distilled water, example 1, example 2, comparative 1, and comparative 2 were calculated, respectively.

Before exposure to cyanides, the mice were placed in a 1-liter barrel, and 1 ml distilled water, example 1, example 2, comparative 1, and comparative 2 were respectively nebulized for mice of each group to inhale. Please refer to FIG. 1B, which illustrates an example of mice inhaling the nebulized dose. The control group inhaled the nebulized distilled water, while the experimental groups inhaled the nebulized example 1, example 2, comparative 1 and comparative 2, respectively. After inhalation for 1 to 2 minutes, the mice were exposed in the cyanide-containing barrel for 45 minutes. The mice were removed from the barrel when death rate reached 20%, and were observed for next 24 hours. Results are recorded in TABLE 2.

As shown in TABLE 2, survival rates of C57BL/6 mice who inhaled example 1 or example 2 were significantly increased when compared with that of control group. Survival rates were increased by 22.0% and 80.0%, respectively, while survival rates of mice inhaling comparative 1 or comparative 2 were insignificantly increased.

**TABLE 2**

| | **Gender** | **age (week)** | **Body weight (g)** | **Cyanide conc. (ppm)** | **Exposure time (min)** | **Increase of survival rate (%)** | |
|---|---|---|---|---|---|---|---|
| **Example 1** | Male | 8 | 21.8 ±0.2 | 185.2 ±5.5 | 24.2 ±7.2 | Control | 0.00 |
| | | | | | | experimental | 22.0 |
| **Example 2** | Male | 8 | 20.6 ±0.2 | 184 | 34 | Control | 0.00 |
| | | | | | | experimental | 80.0 |
| **Comparative 1** | Male | 8 | 20.8 ±0.3 | 184 | 39 | Control | 0.00 |
| | | | | | | experimental | 0.00 |
| **Comparative 2** | Male | 8 | 21.5 ±0.4 | 189.8 ±5.8 | 30.5 ±14.5 | Control | 0.00 |
| | | | | | | experimental | 0.00 |

### Experimental example 2

In the experimental example 2, survival rates of control and experimental groups of ICR mice were compared. The mice were exposed in an environment containing 273 to 320ppm cyanide for 4.5 to 6.0 minutes. Increase of mice survival rates corresponding to each treatment of distilled water, example 1, example 2, comparative 1, and comparative 2 were calculated, respectively.

Before exposure to cyanides, mice of the control group inhaled the nebulized distilled water, while mice of the experimental groups inhaled the nebulized example 1, comparative 1 and comparative 2, respectively. The procedure for inhalation was the same as that in experimental example 1. After inhalation for 1 to 2 minutes, the mice were exposed in cyanide-containing barrel for 5 minutes. The mice were removed from the barrel when death rate reached 20%, and were observed for next 24 hours. Results are recorded in TABLE 3.

As shown in TABLE 3, the survival rate of ICR mice who inhaled example 1 was significantly increased by 50.0% when compared with that of control group. The survival rate of ICR mice who inhaled comparative 1 was only increased by 20.0%, while the survival rate of ICR mice inhaling comparative 2 dropped by 10.0%.

**TABLE 3**

| | **gender** | **age (week)** | **Body weight (g)** | **Cyanide conc. (ppm)** | **Exposure time (min)** | **Increase of survival rate (%)** | |
|---|---|---|---|---|---|---|---|
| **Example 1** | male | 10 to 11 | 37.4 ±2.0 | 273 ±23.7 | 5.3±0.7 | Control | 0.00 |
| | | | | | | experimental | 50.0 |
| **Comparative 1** | male | 8 | 30.0 ±0.2 | 320 | 4.5 | Control | 0.00 |
| | | | | | | experimental | 20.0 |
| **Comparative 2** | male | 8 | 31.3 ±0.2 | 277.2 ±8.1 | 5 | Control | 0.00 |
| | | | | | | experimental | -10.0 |

### Experimental example 3

In experimental example 3, the survival rate of ICR mice after intravenous injection of example 1 was assessed after exposure to cyanide-containing environment. Before exposure to cyanides, mice of control group were injected of distilled water only, while mice of experimental group were injected of 0.1 ml composition from example 1. The mice were exposed in cyanide-containing barrel for 4.5 minutes. The mice were removed from the barrel when death rate reached 20%, and were observed for next 24 hours. Results are recorded in TABLE 4. As shown in TABLE 4, the survival rate of ICR mice after intravenous injection of example 1 was only increased by 20.0% when compared with the survival rate of control group, and the increase of survival rate was far lower than the increase of survival rate in experimental example 2.

**TABLE 4**

| **ICR mice-Intravenous injection** | | | | | | | |
|---|---|---|---|---|---|---|---|
| | **gender** | **age (week)** | **Body weight (g)** | **Cyanide conc. (ppm)** | **Exposure time (min)** | **Increase of survival rate (%)** | |
| **Example 1** | Male | 9 to 10 | 36.5±0.4 | 319.7 | 4.5 | Control | 0.00 |
| | | | | | | experimental | 20.0 |

### Experimental example 4

In experimental example 4, the survival rate of ICR mice after intramuscular injection of example 1 was assessed in after exposure to cyanide-containing environment. Before exposure, mice of control group were injected of distilled water only, while mice of experimental group were injected of 1 ml 5X diluted example 1. The mice were exposed in cyanide-containing barrel for 6.0 minutes. The mice were removed from the barrel when death rate reached 20%, and were observed for next 24 hours. Results are recorded in TABLE 5. As shown in TABLE 5, the survival rate of ICR mice after intramuscular injection of example 1 was insignificantly increased when compared with the survival rate of control group, and the increase of survival rate was similarly far lower than the increase of survival rate in experimental example 2.

**TABLE 5**

| **ICR mice-Intramuscular injection** | | | | | | | |
|---|---|---|---|---|---|---|---|
| | **gender** | **age (week)** | **Body weight (g)** | **Cyanide conc. (ppm)** | **Exposure time (min)** | **Increase of survival rate (%)** | |
| **Example 1** | Male | 9 to 10 | 39.0±1.1 | 305.9 | 6.00 | Control | 0.00 |
| | | | | | | experimental | 0.00 |

### Experimental example 5

In the experimental example 5, survival rates of control and experimental groups of ICR mice were compared. The mice were exposed in an environment containing 460ppm cyanide for 1.0 to 2.0 minutes. Increase of mice survival rates corresponding to each treatment of distilled water and example 1 were calculated, respectively.

Before exposure to cyanides, mice of the control group inhaled only the nebulized distilled water, while mice of the experimental groups inhaled the nebulized example 1. The procedure of inhalation was the same as that in experimental example 1. After inhalation for 1 to 2 minutes, the mice were exposed in cyanide-containing barrel for 5 minutes. The mice were removed from the barrel when death rate reached 20%, and were observed for next 24 hours. Results are recorded in TABLE 6.

As shown in TABLE 6, both groups of mice did not survive in 24 hours after they were removed from the barrel, no matter the mice inhaled distilled water only or the composition from example 1.

**TABLE 6**

| | gender | age (week) | Body weight (g) | Cyanide conc. **(ppm)** | Exposure time (min) | Increase of survival rate **(%)** | |
|---|---|---|---|---|---|---|---|
| **Example 1** | Male | 8 | 30.0± 0.2 | 460 | 1 to 2 | Control | 0.00 (no survival) |
| | | | | | | experimental | 0.00 (no survival) |

With respect to experimental example 1 to 5, the composition for upper respiratory administration provided in the present invention successfully increased survival rates of mice exposed in a cyanide-containing environment after the composition was nebulized and inhaled by the mice. Compared with administration of cyanide antidote alone, a composition comprising cyanide antidote and metal ion chelator significantly increased survival rates of the mice. On the other hand, administration of metal ion chelator alone was not only unable to increase survival rate of the mice, but even decreased mice survival rate after exposure to cyanide-containing environment. Obviously, administration of metal ion chelator alone posed negative effect on mice survival rates. The experimental examples as mentioned above demonstrates that the composition for upper respiratory tract administration not only achieved at protective and curing effect via the cyanide antidote, but also significantly enhanced mice survival rate after exposure to cyanide via a dosage form of composition comprising the metal ion chelator.

The composition for upper respiratory tract administration provided in the present invention overcomes limitations of solution injection in prior arts. The composition can be nebulized and inhaled by a subject in need. The composition enters blood circulation rapidly, which allows the subject to use the composition in general situations, a fireground or an environment having trace amount of cyanides. Moreover, the composition protects the subject from smoke injury at a relatively low dose. The composition not only neutralizes cyanides so as to prevent the subject from acute hypoxia, but also blocks free radical chain reaction after the free radicals enter the body. Oxidative stress on the human body is decreased thereby, so lung tissues and integrity of the upper respiratory tract are both preserved. The composition forms a protective layer on the surfaces of upper respiratory tract or alveoli, and blocks smoke hazardous substances from entering the human body. In other words, the composition provided in the present invention can be inhaled by the subject to protect upper respiratory tract prior to exposure in a fireground or a potential cyanide poisoning environment. The composition can further rapidly enter the human body to neutralize cyanides and to block free radical chain reaction when the subject is exposed in the aforementioned environments. In addition, the composition promotes mucous excretion so that particulate matters in smoke are eliminated from the body. Both first aid of acute toxication and precaution prior to cyanide exposure are taken into considerations.

## Claims

1. A composition for upper respiratory tract administration comprising a cyanide antidote and a metal chelator, and the composition is for use in the cure or protection or prophylaxis of fire injury,
wherein the cyanide antidote is selected from a group consisting of hydroxocobalamin, dicobalt edetate, cobinamide, aquohydroxocobinamide, dinitrocobinamide, methemoglobin, sodium nitrite, amyl nitrite, dimethyl aminophenol, sodium thiosulfate and glutathione,
wherein the metal chelator is selected from a group consisting of deferoxamine, deferiprone and deferasirox, and
wherein the administration is implemented with a dosage form of a spray, a nebulizer, inhaler, or drops.

2. The composition for use according to claim 1, wherein the cyanide antidote is hydroxocobalamin, and wherein the metal ion chelator is deferoxamine.

3. The composition for use according to one of claims 1 to 2, further comprising an expectorant selected from a group consisting of KI, iodinated glycerol, glyceryl guaiacolate, guaifenesin, ambroxol, bromhexine, N-acetylcysteine and lysozyme.

4. The composition for use according to one or more of claims 1 to 3, further comprising a carrier, wherein the carrier comprising ionized water, secondary water, ultra-pure water or buffer solution, wherein the buffer solution having a solute selected from a group consisting of phosphate, tris(hydroxymethyl)aminopropanesulfonic acid, dihydroxyethylglycine, tris(hydroxymethyl)aminomethane, tris(hydroxymethyl)methylglycine, 4-hydroxyethylpiperazineethanesulfonic acid acid, n-tris(hydroxymethyl)methyl-2-aminoethanesulfonic acid, 3-(n-morpholine)ethanesulfonic acid, piperazine-n,n'-bis(2-ethanesulfonic acid), dimethylarsinic acid, sodium citrate and 2-morpholineethanesulfonic acid

5. The composition for use according to one or more of claims 1 to 4, wherein the composition is further used for protection and cure confronting with potential exposure to an environment of high concentration cyanide.

6. The composition for use according to one or more of claims 1 to 5 wherein the composition is administered to the subject in need at a time point until the composition reaches the effective dosage so as to suppress injury upon the subject in need in exposure to toxic gases, wherein the time point is prior to or post exposing the subject in need to the toxic gases.

7. The composition for use according to claim 6, wherein the toxic gases comprise particulate matters, free radicals or hydrogen cyanide.

8. The composition for use according to one or more of claims 1 to 7, wherein the route of administration comprises biological fluid absorption or mucosal absorption.

9. The composition for use according to one or more of claims 1 to 8, wherein the subject in need comprises a mammal selected from a group consisting of human, monkey, mouse, rat, rabbit, canine, cat, bovine, horse, porcine and goat.

10. The composition for use according to one or more of claims 1 to 9, wherein the inhaler is a metered-dose inhaler, a spray inhaler, or a dry powder inhaler.

11. The composition for use according to one or more of claims 1 to 10, wherein the subject in need is human, and wherein the effective dose is reached by administration of the composition at dose of 50 to 300 mg·L⁻¹ for 1 to 2 minutes, and wherein the subject in need is administrated of the composition 3 to 5 minutes before exposure to the toxic gases.

12. The composition for use according to one or more of claims 1 to 13, wherein the composition comprises 5 to 10 parts by weight cyanide antidote and 1 to 5 parts by weight metal ion chelator 5 to 10 parts by weight the expectorant.

## Patentansprüche

1. - Zusammensetzung zur Verabreichung an den oberen Atemtrakt, umfassend ein Cyanid-Antidot und einen Metallchelator, wobei die Zusammensetzung zur Verwendung bei der Heilung oder dem Schutz oder der Prophylaxe von Brandverletzungen ist,
wobei das Cyanid-Antidot ausgewählt ist aus einer Gruppe, bestehend aus Hydroxocobalamin, Dicobalt-Edetat, Cobinamid, Aquohydroxocobinamid, Dinitrocobinamid, Methämoglobin, Natriumnitrit, Amylnitrit, Dimethylaminophenol, Natriumthiosulfat und Glutathion,
wobei der Metallchelator ausgewählt ist aus einer Gruppe, bestehend aus Deferoxamin, Deferipron und Deferasirox, und wobei die Verabreichung in einer Darreichungsform als Spray, Vernebler, Inhalator oder Tropfen implementiert ist.

2. - Zusammensetzung zur Verwendung nach Anspruch 1, wobei das Cyanid-Antidot Hydroxocobalamin ist und wobei der Metallionen-Chelator Deferoxamin ist.

3. - Zusammensetzung zur Verwendung nach einem der Ansprüche 1 bis 2, ferner umfassend ein Expektorans, ausgewählt aus einer Gruppe, bestehend aus KI, iodiertem Glycerol, Glycerylguaiacolat, Guaifenesin, Ambroxol, Bromhexin, N-Acetylcystein und Lysozym.

4. - Zusammensetzung zur Verwendung nach einem oder mehreren der Ansprüche 1 bis 3, ferner umfassend ein Trägermaterial, wobei das Trägermaterial ionisiertes Wasser, Sekundärwasser, ultrareines Wasser oder eine Pufferlösung umfasst, wobei die Pufferlösung einen gelösten Stoff aufweist, der ausgewählt ist aus einer Gruppe, bestehend aus Phosphat, tris(Hydroxymethyl)aminopropansulfonsäure, Dihydroxyethylglycin, tris(Hydroxymethyl)aminomethan, Tris(hydroxymethyl)methylglycin, 4-Hydroxyethylpiperazinethansulfonsäure, n-tris(Hydroxymethyl)methyl-2-aminoethansulfonsäure, 3-(n-Morpholin)ethansulfonsäure, Piperazin-n,n'-bis(2-ethansulfonsäure), Dimethylarsinsäure, Natriumcitrat und 2-Morpholinethansulfonsäure.

5. - Zusammensetzung zur Verwendung nach einem oder mehreren der Ansprüche 1 bis 4, wobei die Zusammensetzung ferner zum Schutz und zur Heilung bei potenzieller Aussetzung an eine Umgebung mit hoher Cyanidkonzentration verwendet wird.

6. - Zusammensetzung zur Verwendung nach einem oder mehreren der Ansprüche 1 bis 5, wobei die Zusammensetzung dem bedürftigen Subjekt zu einem Zeitpunkt verabreicht wird, bis die Zusammensetzung die wirksame Dosierung erreicht, um eine Schädigung des bedürftigen Subjekts bei Aussetzung an toxische Gase zu unterdrücken, wobei der Zeitpunkt vor oder nach der Aussetzung des bedürftigen Subjekts an die toxischen Gase liegt.

7. - Zusammensetzung zur Verwendung nach Anspruch 6, wobei die toxischen Gase Partikel, freie Radikale oder Cyanwasserstoff umfassen.

8. - Zusammensetzung zur Verwendung nach einem oder mehreren der Ansprüche 1 bis 7, wobei der Verabreichungsweg die Absorption über biologische Flüssigkeiten oder mukosale Absorption umfasst.

9. - Zusammensetzung zur Verwendung nach einem oder mehreren der Ansprüche 1 bis 8, wobei das bedürftige Subjekt ein Säugetier ist, ausgewählt aus einer Gruppe, bestehend aus Mensch, Affe, Maus, Ratte, Kaninchen, Hund, Katze, Rind, Pferd, Schwein und Ziege.

10. - Zusammensetzung zur Verwendung nach einem oder mehreren der Ansprüche 1 bis 9, wobei der Inhalator ein Dosieraerosol, ein Sprühinhalator oder ein Trockenpulverinhalator ist.

11. - Zusammensetzung zur Verwendung nach einem oder mehreren der Ansprüche 1 bis 10, wobei das bedürftige Subjekt ein Mensch ist und wobei die wirksame Dosis durch Verabreichung der Zusammensetzung in einer Dosierung von 50 bis 300 mg·L⁻¹ über 1 bis 2 Minuten erreicht wird, und wobei das bedürftige Subjekt 3 bis 5 Minuten vor Aussetzung an die toxischen Gase mit der Zusammensetzung behandelt wird.

12. - Zusammensetzung zur Verwendung nach einem oder mehreren der Ansprüche 1 bis 11, wobei die Zusammensetzung 5 bis 10 Gewichtsanteile Cyanid-Antidot und 1 bis 5 Gewichtsanteile Metallionen-Chelator und 5 bis 10 Gewichtsanteile des Expektorans umfasst.

## Revendications

1. - Composition pour l'administration aux voies respiratoires supérieures, comprenant un antidote de cyanure et un chélateur de métal, et la composition est destinée à être utilisée dans le traitement ou la protection ou la prophylaxie des lésions causées par un incendie,
dans laquelle l'antidote de cyanure est choisi dans un groupe consistant en l'hydroxocobalamine, l'édétate dicobaltique, le cobinamide, l'aquohydroxocobinamide, le dinitrocobinamide, la méthémoglobine, le nitrite de sodium, le nitrite d'amyle, le diméthyl aminophénol, le thiosulfate de sodium et le glutathion,
dans laquelle le chélateur de métal est choisi dans un groupe consistant en la déféroxamine, la défériprone et le déférasirox, et
dans laquelle l'administration est mise en œuvre avec une forme posologique de spray, de nébuliseur, d'inhalateur ou de gouttes.

2. - Composition destinée à être utilisée selon la revendication 1, dans laquelle l'antidote de cyanure est l'hydroxocobalamine, et dans laquelle le chélateur d'ions métalliques est la déféroxamine.

3. - Composition destinée à être utilisée selon l'une des revendications 1 à 2, comprenant en outre un expectorant choisi dans un groupe consistant en KI, la glycérine iodée, la guaïacolate de glycéryle, la guaïfénésine, l'ambroxol, la bromhexine, la N-acétylcystéine et le lysozyme.

4. - Composition destinée à être utilisée selon une ou plusieurs des revendications 1 à 3, comprenant en outre un support, dans laquelle le support comprenant de l'eau ionisée, de l'eau secondaire, de l'eau ultrapure ou une solution tampon, dans laquelle la solution tampon ayant un soluté choisi dans un groupe consistant en le phosphate, l'acide tris(hydroxyméthyl)aminopropanesulfonique, la dihydroxyéthylglycine, le tris(hydroxyméthyl)aminométhane, la tris(hydroxyméthyl)méthylglycine, l'acide 4-hydroxyéthylpipérazine éthane sulfonique, l'acide n-tris(hydroxyméthyl)méthyl-2-aminoéthanesulfonique, l'acide 3-(n-morpholine)éthanesulfonique, l'acide pipérazine-n,n'-bis(2-éthanesulfonique), l'acide diméthylarsinique, le citrate de sodium et l'acide 2-morpholineéthanesulfonique.

5. - Composition destinée à être utilisée selon une ou plusieurs des revendications 1 à 4, dans laquelle la composition est en outre utilisée pour la protection et le traitement en cas d'exposition potentielle à un environnement à forte concentration en cyanure.

6. - Composition destinée à être utilisée selon une ou plusieurs des revendications 1 à 5, dans laquelle la composition est administrée à un sujet en ayant besoin à un moment jusqu'à ce que la composition atteigne une dose efficace afin de supprimer les lésions subies par le sujet en ayant besoin lors d'une exposition à des gaz toxiques, dans laquelle le moment est avant ou après l'exposition du sujet en ayant besoin aux gaz toxiques.

7. - Composition destinée à être utilisée selon la revendication 6, dans laquelle les gaz toxiques comprennent des matières particulaires, des radicaux libres ou du cyanure d'hydrogène.

8. - Composition destinée à être utilisée selon une ou plusieurs des revendications 1 à 7, dans laquelle la voie d'administration comprend l'absorption de fluides biologiques ou l'absorption par muqueuse.

9. - Composition destinée à être utilisée selon une ou plusieurs des revendications 1 à 8, dans laquelle le sujet en ayant besoin comprend un mammifère choisi dans un groupe consistant en un être humain, un singe, une souris, un rat, un lapin, un chien, un chat, un bovin, un cheval, un porc et une chèvre.

10. - Composition destinée à être utilisée selon une ou plusieurs des revendications 1 à 9, dans laquelle l'inhalateur est un inhalateur doseur, un inhalateur à spray ou un inhalateur de poudre sèche.

11. - Composition destinée à être utilisée selon une ou plusieurs des revendications 1 à 10, dans laquelle le sujet en ayant besoin est un être humain, et dans laquelle la dose efficace est atteinte par l'administration de la composition à une dose de 50 à 300 mg·L⁻¹ pendant 1 à 2 minutes, et dans laquelle le sujet en ayant besoin reçoit la composition 3 à 5 minutes avant l'exposition aux gaz toxiques.

12. - Composition destinée à être utilisée selon une ou plusieurs des revendications 1 à 11, dans laquelle la composition comprend 5 à 10 parties en poids d'antidote de cyanure et 1 à 5 parties en poids de chélateur d'ions métalliques et 5 à 10 parties en poids d'expectorant.
